# EUROPEAN PATENT SPECIFICATION

(11) **EP 4 049 630 B1**
(45) Date of publication and mention of the grant of the patent: **27.03.2024**
(21) Application number: 22158889.0
(22) Date of filing: 25.02.2022
(51) Int. Cl.: A61F 2/46, A01N 1/02, A61J 1/10

(54) **CONTAINER FOR BONE MATERIAL STORAGE, MIXING AND DELIVERY**
BEHÄLTER ZUM LAGERN, MISCHEN UND ABGEBEN VON KNOCHENMATERIAL
CONTENEUR POUR LE STOCKAGE, LE MÉLANGE ET LA DISTRIBUTION DE MATÉRIAU OSSEUX

(30) Priority: 26.02.2021 US 202163154194 P; 22.02.2022 US 202217677421
(43) Date of publication of application: 31.08.2022
(73) Proprietor: Warsaw Orthopedic, Inc., Warsaw, IN 46582 (US)
(72) Inventor: RISTER, Jason A., Minneapolis, MN (US); RHODES, Cheyenne, Minneapolis, MN (US); RAJAGOPALAN, Senapathy, Minneapolis, MN (US); SHIMKO, Daniel, Minneapolis, MN (US)
(74) Representative: Viering, Jentschura & Partner mbB Patent- und Rechtsanwälte

(56) References cited:
- WO-A1-03/008285
- WO-A1-2014/130953
- WO-A1-2019/204268
- US-A1- 2020 008 921

## Description

### BACKGROUND

The use of bone material including natural bone and bone substitute materials for filling a bone repair site is often used in orthopedic medicine. While bone wounds can regenerate without the formation of scar tissue, fractures and other orthopedic injuries take a long time to heal, during which time the bone is unable to support physiologic loading without the use of implantable medical devices. Metal pins, screws, rods, plates and meshes are frequently required to replace the mechanical functions of injured bone. However, metal is significantly stiffer than bone. Use of metal implants may result in decreased bone density around the implant site due to stress shielding. Physiologic stresses and corrosion may cause metal implants to fracture. Unlike bone, which can heal small cracks through remodeling to prevent more extensive damage and failure, damaged metal implants can only be replaced or removed. The natural cellular healing and remodeling mechanisms of the body coordinate removal of bone and bone grafts by osteoclast cells and formation of bone by osteoblast cells.

The rapid and effective repair of bone defects caused by injury, disease, wounds, or surgery has long been a goal of orthopedic surgery. Toward this end, a number of materials have been used or proposed for use in the repair of bone defects. The biological, physical, and mechanical properties of the materials are among the major factors influencing their suitability and performance in various orthopedic applications.

Autologous cancellous bone (ACB) has long been considered the gold standard for bone grafts. ACB includes osteogenic cells, which have the potential to assist in bone healing, is nonimmunogenic, and has structural and functional characteristics that are appropriate for a healthy recipient. Some people do not have adequate amounts of ACB for harvesting. These people include, for example, older people and people who have had previous surgeries. A majority of people, however, do have adequate amounts of ACB for harvesting. There may nevertheless be reluctance to harvest ACB because of pain at the harvest site and potential donor site morbidity.

Conventionally, bone tissue regeneration is achieved by filling a bone defect with a bone material, for example, a bone graft. Over time, the bone graft is incorporated by the host and new bone remodels the bone graft. Bone material can include bone from the patient's own body or artificial, synthetic, or natural substitute bone material. However, handling of the bone material, including mixing it with different components and dispensing it can be difficult to reduce waste and introduction of contaminants into the bone material can occur.

To deliver the bone material to the bone defect, often times the bone material is mixed with liquid or a therapeutic agent, powder, fiber or granular material. Mixing devices that are currently available can be cumbersome to use and do not allow uniform and easy mixing of material. Mixing of dry bone grafts with fluids to hydrate can be especially difficult and frustrating as mechanical force and/or additional instrumentation may be necessary to efficiently compress, mix, stir, or knead, the materials to evenly hydrate or distribute the fluid. Further, transfer of bone material to the delivery device is often done by crude packing of the bone delivery device and there is unwanted spillage of bone material out of the device, which may increase the risk of contamination of the bone material. Mixing and transfer of bone graft materials often is necessary to create an implantable construct and treat a patient in need of bone graft. This process can occur aseptically in a surgical environment. Often times material can be transferred between vessels to completely mix materials to create the implantable construct. When this is done by one person, container and delivery system designs can consider the challenges of vessel and instrument handling when only two hands from one surgical staff member are available to complete the task. Currently available containers and delivery devices often lack features that facilitate easy aseptic transfer between vessels and lack precision in measuring the bone material for delivery to the target bone defect.

It would therefore be beneficial to provide a container for effectively and efficiently, staging, measuring, mixing and dispensing of bone material. Further, a container for dispensing bone material that allows for easy loading and mixing of the bone and fluid inside the container and reduces the risk of contamination and spillage of bone material from the container would also be beneficial.

Further to the above, a container and method for mixing bone material according to the preamble of the independent claims are known from, e.g., WO 2014/130953 A1. Further containers and methods for mixing bone material are known from, e.g., US 2020/008921 A1 and WO 2019/204268 A1.

### SUMMARY

The present invention is set out in the appended claims and provides a container for mixing bone material according to claim 1 and a method of mixing bone material according to claim 13. Further advantageous embodiments are described in the dependent claims. A container for mixing bone material according to the present invention allows for easy staging, measuring, mixing and dispensing of the bone material. The bone material container allows easier loading of the container, which reduces the risk of contamination and spillage of bone material. In some embodiments, the bone material container allows uniform mixing and measuring of the bone material, reducing clogging and friction resistance in the bone material container.

While multiple embodiments are disclosed, still other embodiments of the present disclosure will become apparent to those skilled in the art from the following detailed description. As will be apparent, the disclosure is capable of modifications in various obvious aspects, all without departing from the scope of the present disclosure. Accordingly, the detailed description is to be regarded as illustrative in nature and not restrictive.

### BRIEF DESCRIPTION OF THE FIGURES

In part, other aspects, features, benefits and advantages of the embodiments will be apparent with regard to the following description, appended claims and accompanying figures where:
FIG. 1 depicts a perspective view of a container for staging, measuring, mixing and/or dispensing bone material according to an aspect of the present invention;
FIG. 2 depicts a partial view of the container illustrated in FIG. 1 in an open configuration with bone material partially being filled in the container;
FIG. 3 depicts a perspective view of a syringe used to place fluid in the container illustrated at FIG. 1 at the port for mixing with the bone material.
FIG. 4A depicts a perspective view of the container of FIG. 1 illustrating the use of a staging surface with measuring features when the locking member is closed.
FIG. 4B depicts a perspective view of the container of FIG. 1 illustrating the use of the staging surface with measuring features when the locking member is open.
FIG. 5 depicts a perspective view of the container of FIG. 1 illustrating the upper layer being peeled away from the lower layer of the covering of the container.
FIG. 6A depicts a perspective view of the container of FIG. 1 illustrating the removal of the port along tear lines positioned adjacent to the container port.
FIG. 6B depicts a perspective view of the container of FIG. 1 illustrating the removal of the staging surface with measuring features along tear lines positioned adjacent the locking member.
FIG. 7 depicts a perspective view of the container of FIG. 1 illustrating bone material mixed with fluid being dispensed from the container by rolling it from the distal end to its proximal end.

It is to be understood that the figures are not drawn to scale. Further, the relation between objects in a figure may not be to scale and may in fact have a reverse relationship as to size. The figures are intended to bring understanding and clarity to the structure of each object shown, and thus, some features may be exaggerated in order to illustrate a specific feature of a structure.

### DETAILED DESCRIPTION

### Definitions

It is noted that, as used in this specification and the appended claims, the singular forms "a," "an," and "the," include plural referents unless expressly and unequivocally limited to one referent. For example, reference to "a container" includes one, two, three or more containers.

The term "allograft" refers to a graft of tissue obtained from a donor of the same species as, but with a different genetic make-up from, the recipient, as a tissue transplant between two humans.

The term "autologous" refers to being derived or transferred from the same individual's body, such as for example an autologous bone marrow transplant.

The term "xenograft" refers to tissue or organs from an individual of one species transplanted into or grafted onto an organism of another species, genus, or family.

The term "mammal" refers to organisms from the taxonomy class "mammalian," including, but not limited to, humans; other primates, such as chimpanzees, apes, orangutans and monkeys; rats, mice, cats, dogs, cows, horses, etc.

The term "patient" refers to a biological system to which a treatment can be administered. A biological system can include, for example, an individual cell, a set of cells (e.g., a cell culture), an organ, or a tissue. Additionally, the term "patient" can refer to animals, including, without limitation, humans.

The term "bone material" includes natural and/or inorganic material such as, for example, inorganic ceramic and/or bone substitute material. The bone material can also include natural bone material such as, for example, bone which is cortical, cancellous or cortico-cancellous of autogenous, allogenic, xenogenic, or transgenic origin. In some embodiments, bone material can include demineralized bone material such as, for example, substantially demineralized bone material, partially demineralized bone material, or fully demineralized bone material.

"Demineralized" as used herein, refers to any material generated by removing mineral material from tissue, e.g., bone tissue. In certain embodiments, the demineralized compositions described herein include preparations containing less than 5% calcium and preferably less than 1% calcium by weight. Partially demineralized bone (e.g., preparations with greater than 5% calcium by weight but containing less than 100% of the original starting amount of calcium) is also considered within the scope of the application. In some examples, demineralized bone has less than 95% of its original mineral content.

In some examples, demineralized bone has less than 95% of its original mineral content. In some examples, demineralized bone has less than 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6 and/or 5% of its original content. In some examples, "demineralized" is intended to encompass such expressions as "substantially demineralized," "superficially demineralized," "partially demineralized," "surface demineralized," and "fully demineralized."

"Partially demineralized" is intended to encompass "surface demineralized." "Partially demineralized bone" is intended to refer to preparations with greater than 5% calcium by weight but containing less than 100% of the original starting amount of calcium. In some examples, partially demineralized comprises 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 and/or 99% of the original starting amount of calcium.

In some examples, the demineralized bone may be surface demineralized from about 1-99%. In some examples, the demineralized bone is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 and/or 99% surface demineralized. In various examples, the demineralized bone may be surface demineralized from about 15-25%. In some examples, the demineralized bone is 15, 16, 17, 18, 19, 20, 21, 22, 23, 24 and/or 25% surface demineralized.

"Superficially demineralized" as used herein, refers to bone-derived elements possessing at least about 90 weight percent of their original inorganic mineral content, the expression "partially demineralized" as used herein refers to bone-derived elements possessing from about 8 to about 90 weight percent of their original inorganic mineral content and the expression "fully demineralized" as used herein refers to bone containing less than 8% of its original mineral context.

"Demineralized bone matrix" as used herein, refers to any material generated by removing mineral material from bone tissue. In preferred embodiments, the DBM compositions as used herein include preparations containing less than 5% calcium and preferably less than 1% calcium by weight.

"Biocompatible" as used herein, refers to materials that, upon administration in vivo, do not induce undesirable long-term effects.

"Osteoconductive" as used herein, refers to the ability of a non-osteoinductive substance to serve as a suitable template or substance along which bone may grow.

"Osteogenic", as used herein, refers to the ability of an agent, material, or implant to enhance or accelerate the growth of new bone tissue by one or more mechanisms such as osteogenesis, osteoconduction, and/or osteoinduction.

"Osteoinductive" as used herein, refers to the quality of being able to recruit cells from the host that have the potential to stimulate new bone formation. Any material that can induce the formation of ectopic bone in the soft tissue of an animal is considered osteoinductive. For example, most osteoinductive materials induce bone formation in athymic rats when assayed according to the method of Edwards et al., "Osteoinduction of Human Demineralized Bone: Characterization in a Rat Model," Clinical Orthopaedics & Rel. Res., 357:219-228, December 1998.

The terms "upper", "lower", "top", "bottom", "side", "proximal", "distal" and so forth have been used herein merely for convenience to describe the present invention and its parts as oriented in the drawings. It is to be understood, however, that these terms are in no way limiting to the disclosure since the delivery systems described herein may obviously be disposed in different orientations when in use.

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding the numerical ranges and parameters set forth herein, the broad scope of the invention is an approximation; the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, *e.g.,* 5.5 to 10.

Reference will now be made in detail to certain embodiments of the disclosure, examples of which are illustrated in the accompanying figures. While the disclosure will be described in conjunction with the illustrated embodiments, it will be understood that they are not intended to limit the disclosure to those embodiments. On the contrary, the disclosure is intended to cover all alternatives and modifications that may be included within the disclosure as defined by the appended claims.

The headings below are not meant to limit the disclosure in any way; embodiments under anyone heading may be used in conjunction with embodiments under any other heading.

### Bone Material

According to the invention as defined in claim 1, there is a container for staging, measuring, mixing and/or dispensing bone material that allows measuring and easy mixing of bone material. The container allows for easier loading of the bone material, which reduces the risk of contamination and spillage of bone material. In some embodiments, the container for mixing and/or dispensing bone material allows uniform mixing and measuring of the bone material, reducing clogging and friction resistance in the bone material container. The bone material can be in granular, paste, putty or powder forms.

In some embodiments, the bone material can be made from natural bone and/or synthetic bone. The bone material can include ceramic, collagen, allograft bone, autograft bone, demineralized bone matrix fibers, demineralized bone powder, demineralized bone chips or a combination thereof.

In various embodiments, the bone material may be particulated such as, for example, in bone chips, powder or fiber form. In some embodiments, the bone material is in a powder or a wet form and has a particle size of 250 microns or less. In some embodiments, the bone material has a particle size of 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248 and/or 250 microns. In some embodiments, the bone material is demineralized bone (DBM).

If the bone is demineralized, the bone may be made into a particulate before, during or after demineralization. In some embodiments, the bone may be monolithic and may not be a particulate.

The bone may be milled and ground or otherwise processed into particles of an appropriate size before or after demineralization. The particles may be particulate (for example, powder) or fibrous. The terms milling or grinding are not intended to be limited to production of particles of a specific type and may refer to production of particulate or fibrous particles. In certain embodiments, the particle size may be greater than 25 microns, such as ranging from about 25 to about 250 microns, or from about 25 to about 200 microns or from about 25 to about 150 microns.

After grinding, the bone particles may be sieved to select those particles of a desired size. In certain embodiments, the particles may be sieved though a 25-micron sieve, a 50-micron sieve, a 75-micron sieve, a 100-micron sieve, a 125-micron sieve, a 150-micron sieve, a 175-micron sieve and/or a 200-micron sieve.

In some embodiments, the bone material comprises DBM and/or mineralized bone. In some embodiments, the size of the bone material is less than 25, 50, 75, 100, 125, 150, 175, 200 or 250 microns.

Following shaving, milling or other technique whereby they are obtained, the bone material is subjected to demineralization in order to reduce its inorganic content to a very low level, in some embodiments, to not more than about 5% by weight of residual calcium and to not more than about 1% by weight of residual calcium. Demineralization of the bone material ordinarily results in its contraction to some extent.

Bone used in the methods described herein may be autograft, allograft, or xenograft. In various embodiments, the bone may be cortical bone, cancellous bone, or cortico-cancellous bone. While specific discussion is made herein to demineralized bone matrix, bone matrix treated in accordance with the teachings herein may be non-demineralized, demineralized, partially demineralized, or surface demineralized. This discussion applies to demineralized, partially demineralized, and surface demineralized bone matrix. In one embodiment, the demineralized bone is sourced from bovine or human bone. In another embodiment, demineralized bone is sourced from human bone. In one embodiment, the demineralized bone is sourced from the patient's own bone (autogenous bone). In another embodiment, the demineralized bone is sourced from a different animal (including a cadaver) of the same species (allograft bone).

Any suitable manner of demineralizing the bone may be used. Demineralization of the bone material can be conducted in accordance with known conventional procedures. For example, in a preferred demineralization procedure, the bone materials useful for the implantable composition of this application are subjected to an acid demineralization step that is followed by a defatting/disinfecting step. The bone material is immersed in acid over time to effect its demineralization. Acids which can be employed in this step include inorganic acids such as hydrochloric acid and organic acids such as peracetic acid, acetic acid, citric acid, or propionic acid. The depth of demineralization into the bone surface can be controlled by adjusting the treatment time, temperature of the demineralizing solution, concentration of the demineralizing solution, agitation intensity during treatment, and other applied forces such as vacuum, centrifuge, pressure, and other factors such as known to those skilled in the art. Thus, in various embodiments, the bone material may be fully demineralized, partially demineralized, or surface demineralized.

After acid treatment, the bone is rinsed with sterile water for injection, buffered with a buffering agent to a final predetermined pH and then finally rinsed with water for injection to remove residual amounts of acid and buffering agent or washed with water to remove residual acid and thereby raise the pH. Following demineralization, the bone material is immersed in solution to effect its defatting. A defatting/disinfectant solution is an aqueous solution of ethanol, the ethanol being a good solvent for lipids and the water being a good hydrophilic carrier to enable the solution to penetrate more deeply into the bone. The aqueous ethanol solution also disinfects the bone by killing vegetative microorganisms and viruses. Ordinarily at least about 10 to 40 weight percent by weight of water (i.e., about 60 to 90 weight percent of defatting agent such as alcohol) should be present in the defatting/disinfecting solution to produce optimal lipid removal and disinfection within the shortest period of time. The concentration range of the defatting solution is from about 60 to 85 weight percent alcohol or about 70 weight percent alcohol.

Further in accordance with this application, the DBM material can be used immediately for preparation of the bone implant or it can be stored under aseptic conditions, advantageously in a critical point dried state prior to such preparation. In one embodiment, the bone material can retain some of its original mineral content such that the composition is rendered capable of being imaged utilizing radiographic techniques.

In various embodiments, this application also provides bone matrix compositions comprising critical point drying (CPD) fibers. DBM includes the collagen matrix of the bone together with acid insoluble proteins including bone morphogenic proteins (BMPs) and other growth factors. It can be formulated for use as granules, gels, sponge material or putty and can be freeze-dried for storage. Sterilization procedures used to protect from disease transmission may reduce the activity of beneficial growth factors in the DBM. DBM provides an initial osteoconductive matrix and exhibits a degree of osteoinductive potential, inducing the infiltration and differentiation of osteoprogenitor cells from the surrounding tissues.

DBM preparations have been used for many years in orthopedic medicine to promote the formation of bone. For example, DBM has found use in the repair of fractures, in the fusion of vertebrae, in joint replacement surgery, and in treating bone destruction due to underlying disease such as rheumatoid arthritis. DBM is thought to promote bone formation in vivo by osteoconductive and osteoinductive processes. The osteoinductive effect of implanted DBM compositions is thought to result from the presence of active growth factors present on the isolated collagen-based matrix. These factors include members of the TGF-β, IGF, and BMP protein families. Particular examples of osteoinductive factors include TGF-β, IGF-1, IGF-2, BMP-2, BMP-7, parathyroid hormone (PTH), and angiogenic factors. Other osteoinductive factors such as osteocalcin and osteopontin are also likely to be present in DBM preparations as well. There are also likely to be other unnamed or undiscovered osteoinductive factors present in DBM.

In various embodiments, the DBM provided in this application can be prepared from elongated bone fibers which have been subjected to critical point drying (CPD). The elongated CPD bone fibers employed in this application are generally characterized as having relatively high average length to average width ratios, also known as the aspect ratio. In various embodiments, the aspect ratio of the elongated bone fibers is at least from about 50:1 to at least about 1000: 1. Such elongated bone fibers can be readily obtained by any one of several methods, for example, by milling or shaving the surface of an entire bone or relatively large section of bone.

In other embodiments, the length of the fibers can be at least about 3.5 cm and average width from about 20 mm to about 1 cm. In various embodiments, the average length of the elongated fibers can be from about 3.5 cm to about 6.0 cm and the average width from about 20 mm to about 1 cm. In other embodiments, the elongated fibers can have an average length from about 4.0 cm to about 6.0 cm and an average width from about 20 mm to about 1 cm.

In yet other embodiments, the diameter or average width of the elongated fibers is, for example, not more than about 1.00 cm, not more than 0.5 cm or not more than about 0.01 cm. In still other embodiments, the diameter or average width of the fibers can be from about 0.01 cm to about 0.4 cm or from about 0.02 cm to about 0.3 cm.

In another embodiment, the aspect ratio of the fibers can be from about 50:1 to about 950:1, from about 50:1 to about 750:1, from about 50:1 to about 500:1, from about 50:1 to about 250:1; or from about 50:1 to about 100:1. Fibers according to this disclosure can have an aspect ratio from about 50:1 to about 1000: 1, from about 50:1 to about 950:1, from about 50:1 to about 750:1, from about 50:1 to about 600:1, from about 50:1 to about 350:1, from about 50:1 to about 200:1, from about 50:1 to about 100:1, or from about 50:1 to about 75:1.

In some embodiments, the chips to fibers ratio is about 90:10, 80:20, 75:25, 70:30, 60:40, 50:50, 40:60, 30:70, 25:75, 20:80 and/or 10:90. In various embodiments, a surface demineralized chips to fibers ratio is about 90:10, 80:20, 75:25, 70:30, 60:40, 50:50, 40:60, 30:70, 25:75, 20:80 and/or 10:90. In some embodiments, a surface demineralized chips to fully demineralized fibers ratio is about 90:10, 80:20, 75:25, 70:30, 60:40, 50:50, 40:60, 30:70, 25:75, 20:80 and/or 10:90.

In some embodiments, the DBM fibers have a thickness of about 0.5-4 mm. In various embodiments, the DBM fibers have a thickness of about 0.5, 0.6, 0.7, 0.8, 0.9, 1, 1.5, 2, 2.5, 3, 3.5 and/or 4 mm. In various embodiments, the ratio of DBM fibers to DBM powder is about 40:60 to about 90:10 W/W, W/V or V/V. In some embodiments, the ratio of mineralized bone fibers to DBM powder is about 25:75 to about 75:25 W/W, W/V or V/V. In various embodiments, the bone implant comprises DBM fibers and mineralized fibers in a ratio of 40:60 to about 90:10 W/W, W/V or V/V. In some embodiments, the DBM fibers to DBM powder ratio, mineralized bone fibers to DBM powder ratio and/or the DBM fibers and mineralized fibers ratio is from 5:95 to about 95:5 W/W, W/V or V/V. In some embodiments, the DBM fibers to DBM powder ratio, mineralized bone fibers to DBM powder ratio and/or the DBM fibers and mineralized fibers ratio is 5:95, 10:90, 15:85, 20:80, 25:75, 30:70, 35:65, 40:60, 45:55, 50:50, 55:45, 60:40, 65:35, 70:30, 75:25, 80:20, 85:15, 90:10 and/or 95:5 W/W, W/V or V/V.

In some embodiments, the bone material comprises demineralized bone material comprising demineralized bone, fibers, powder, chips, triangular prisms, spheres, cubes, cylinders, shards or other shapes having irregular or random geometries. These can include, for example, "substantially demineralized," "partially demineralized," or "fully demineralized" cortical and/or cancellous bone. These also include surface demineralization, where the surface of the bone construct is substantially demineralized, partially demineralized, or fully demineralized, yet the body of the bone construct is fully mineralized.

In various embodiments, the bone material comprises fully DBM fibers and surface demineralized bone chips. In some embodiments, the ratio of fully DBM fibers to surface demineralized bone chips is from 5:95 to about 95:5 fibers to chips. In some embodiments, the ratio of fully DBM fibers to surface demineralized bone chips is 5:95, 10:90, 15:85, 20:80, 25:75, 30:70, 35:65, 40:60, 45:55, 50:50, 55:45, 60:40, 65:35, 70:30, 75:25, 80:20, 85:15, 90:10 and/or 95:5 fibers to chips. In various embodiments, the fully DBM fibers have a thickness of about 0.5-4 mm. In various embodiments, the fully DBM fibers have a thickness of about 0.5, 0.6, 0.7, 0.8, 0.9, 1, 1.5, 2, 2.5, 3, 3.5 and/or 4 mm.

In various embodiments, the fibers and/or the powder is surface DBM. In some embodiments, the fibers and/or the powder is surface DBM cortical allograft. In various embodiments, surface demineralization involves surface demineralization to at least a certain depth. For example, the surface demineralization of the allograft can be from about 0.25 mm, 0.5 mm, 1 mm, 1.5 mm, 2.0 mm, 2.5 mm, 3.0 mm. 3.5 mm, 4 mm, 4.5 mm, to about 5 mm. The edges of the bone fibers and/or powder may further be machined into any shape or to include features such as grooves, protrusions, indentations, etc., to help improve fit and limit any movement or micromotion to help fusion and/or osteoinduction to occur.

To prepare the osteogenic DBM, a quantity of fibers is combined with a biocompatible carrier to provide a demineralized bone matrix.

DBM typically is dried, for example via lyophilization or solvent drying, to store and maintain the DBM in active condition for implantation. Moreover, each of these processes is thought to reduce the overall surface area structure of bone. As may be appreciated, the structural damage of the exterior surface reduces the overall surface area. Physical alterations to the surface and reduction in surface area can affect cell attachment, mobility, proliferation, and differentiation. The surface's affinity for growth factors and release kinetics of growth factors from the surface may also be altered.

Accordingly, in some examples, methods for drying bone to store and maintain the bone in active condition for implantation that maintains or increases the surface area of the bone are provided. In one embodiment, the bone matrix is treated using a critical point drying technique, thereby reducing destruction of the surface of the bone. While specific description is made to critical point drying, it is to be appreciated that, in alternative embodiments, super critical point treatment may be used. In various embodiments utilizing CPD, a percentage of collagen fibrils on the surface of the bone are non-denatured after drying to a residual moisture content of approximately 15% or less. In some embodiments, after drying, the bone matrix has a residual moisture content of approximately 8% or less. In some embodiments, after drying, the bone matrix has a residual moisture content of approximately 6% or less. In some embodiments, after drying, the bone matrix has a residual moisture content of approximately 3% or less.

Evaporative drying and freeze drying of specimens can cause deformation and collapse of surface structures, leading to a decrease in surface area. Without wishing to be bound by a particular theory, this deformation and structure is thought to occur because as a substance crosses the boundary from liquid to gas, the substance volatilizes such that the volume of the liquid decreases. As this happens, surface tension at the solid-liquid interface pulls against any structures to which the liquid is attached. Delicate surface structures tend to be broken apart by this surface tension. Such damage may be caused by the effects of surface tension on the liquid/gas interface. Critical point drying is a technique that avoids effects of surface tension on the liquid/gas interface by substantially preventing a liquid/gas interface from developing. Critical point or supercritical drying does not cross any phase boundary, instead passing through the supercritical region, where the distinction between gas and liquid ceases to apply. As a result, materials dehydrated using critical point drying are not exposed to damaging surface tension forces. When the critical point of the liquid is reached, it is possible to pass from liquid to gas without abrupt change in state. Critical point drying can be used with bone matrices to phase change from liquid to dry gas without the effects of surface tension. Accordingly, bone dehydrated using critical point drying can retain or increase at least some of the surface structure and therefore the surface area.

In some embodiments, critical point drying is carried out using carbon dioxide. However, other mediums such as Freon, including Freon 13 (chlorotrifluoromethane), may be used. Generally, fluids suitable for supercritical drying include carbon dioxide (critical point 304.25 K at 7.39 MPa or 31.1° C at 1072 psi or 31.2° C and 73.8 bar) and Freon (about 300 K at 3.5-4 MPa or 25 to 30° C at 500-600 psi). Nitrous oxide has similar physical behavior to carbon dioxide but is a powerful oxidizer in its supercritical state. Supercritical water is also a powerful oxidizer, partly because its critical point occurs at such a high temperature (374° C) and pressure (3212 psi/647K and 22.064 MPa).

In some embodiments, the bone may be pretreated to remove water prior to critical point drying. Thus, in accordance with one embodiment, bone matrix is dried using carbon dioxide in (or above) its critical point status. After demineralization, bone matrix samples (in water) may be dehydrated to remove residual water content. Such dehydration may be, for example, through a series of graded ethanol solutions (for example, 20%, 50%, 70%, 80%, 90%, 95%, 100% ethanol in deionized water). In some embodiments, penetrating the tissue with a graded series of ethanol solutions or alcohols may be accomplished in an automated fashion. For example, pressure and vacuum could be used to accelerate penetration into the tissue.

In some embodiments, the bone material comprises demineralized bone matrix fibers and demineralized bone matrix chips in a 30:60 ratio. In some embodiments, the bone material comprises demineralized bone matrix fibers and demineralized bone matrix chips in a ratio of 25:75 to about 75:25 fibers to chips.

In some embodiments, the bone material can be an inorganic material, such as an inorganic ceramic and/or bone substitute material. Exemplary inorganic materials or bone substitute materials include but are not limited to aragonite, dahlite, calcite, brushite, amorphous calcium carbonate, vaterite, weddellite, whewellite, struvite, urate, ferrihydrate, francolite, monohydrocalcite, magnetite, goethite, dentin, calcium carbonate, calcium sulfate, calcium phosphosilicate, sodium phosphate, calcium aluminate, calcium phosphate, hydroxyapatite, alphatricalcium phosphate, dicalcium phosphate, β-tricalcium phosphate, tetracalcium phosphate, amorphous calcium phosphate, octacalcium phosphate, BIOGLASS^{™} fluoroapatite, chlorapatite, magnesium-substituted tricalcium phosphate, carbonate hydroxyapatite, substituted forms of hydroxyapatite (e.g., hydroxyapatite derived from bone may be substituted with other ions such as fluoride, chloride, magnesium sodium, potassium, etc.), or combinations or derivatives thereof.

In some embodiments, the bone material can comprise mineral particles, which comprise tricalcium phosphate and hydroxyapatite in a ratio of about 80:20 to about 90:10. In some embodiments, the mineral particles can comprise tricalcium phosphate and hydroxyapatite in a ratio of about 70:30 to about 95:5. In some embodiments, the mineral particles can comprise tricalcium phosphate and hydroxyapatite in a ratio of about 85:15.

In some embodiments, the bone material may be seeded with harvested bone cells and/or bone tissue, such as for example, cortical bone, autogenous bone, allogenic bones and/or xenogeneic bone while it is mixed.

Suitable bone material for use with the container of the current application includes, for example, Magnifuse^{™} Bone Graft, Grafton^{™} DBM available in DBM fibers, powder, granules, gels, matrices, strips, putties or pastes, which are available from Medtronic Sofamor Danek, Memphis, Tenn., USA.

In some embodiments, the bone material may be mixed with one or more therapeutic agents, for example, an anti-inflammatory agent, an analgesic agent, an osteoinductive growth factor, an antimicrobial agent or a combination thereof. Osteoinductive agents include one or more members of the family of Bone Morphogenetic Proteins ("BMPs"). BMPs are a class of proteins thought to have osteoinductive or growth-promoting activities on endogenous bone tissue, or function as pro-collagen precursors.

BMPs utilized as osteoinductive agents comprise one or more of BMP-1; BMP-2; BMP-3; BMP-4; BMP-5; BMP-6; BMP-7; BMP-8; BMP-9; BMP-10; BMP-11; BMP-12; BMP-13; BMP-15; BMP-16; BMP-17; or BMP-18; as well as any combination of one or more of these BMPs, including full length BMPs or fragments thereof, or combinations thereof, either as polypeptides or polynucleotides encoding the polypeptide fragments of all of the recited BMPs. The isolated BMP osteoinductive agents may be administered as polynucleotides, polypeptides, full length protein or combinations thereof.

Indeed, the osteoinductive factors are the recombinant human bone morphogenetic proteins (rhBMPs) because they are available in unlimited supply and do not transmit infectious diseases. In some embodiments, the bone morphogenetic protein is a rhBMP-2, rhBMP-4, rhBMP-7, or heterodimers thereof. Recombinant BMP-2 can be used at a concentration of about 0.4 mg/mL to about 10.0 mg/mL, preferably about 1.5 mg/mL.

The bone material may include or be mixed with one or more members from the TGF-β superfamily. For example, the matrix may include AMH, ARTN, GDF1, GDF10, GDF11, GDF15, GDF2, GDF3, GDF3A, GDF5, GDF6, GDF7, GDF8, GDF9, GDNF, INHA, INHBA, INHBB, INHBC, INHBE, LEFTY1, LEFTY2, MSTN, NODAL, NRTN, PSPN, TGFB1, TGFB2, TGFB3, FGF, basic FGF, VEGF, insulin-like growth factor, EGF, PDGF, nerve growth factor or combinations thereof.

The bone material may include or be mixed with a therapeutic agent including, but not limited to, IL-1 inhibitors, such Kineret^{®} (anakinra), which is a recombinant, non-glycosylated form of the human interleukin-1 receptor antagonist (II,-1Ra), or AMG 108, which is a monoclonal antibody that blocks the action of IL-1. The bone material may include or be mixed with therapeutic agents including excitatory amino acids such as glutamate and aspartate, antagonists or inhibitors of glutamate binding to NMDA receptors, AMPA receptors, and/or kainate receptors. The bone material may include or be mixed with therapeutic agents to reduce inflammation including but not limited to interleukin-1 receptor antagonists, thalidomide (a TNF-α release inhibitor), thalidomide analogues (which reduce TNF-α production by macrophages), quinapril (an inhibitor of angiotensin II, which upregulates TNF-α), interferons such as IL-11 (which modulate TNF-α receptor expression), or aurin-tricarboxylic acid (which inhibits TNF-α).

The bone material may include or be mixed with a therapeutic agent including, but not limited to, an analgesic agent. Examples of analgesic agents include, but are not limited to, acetaminophen, tramadol, lidocaine, bupivacaine, ropivacaine, opioid analgesics such as buprenorphine, butorphanol, dextromoramide, dezocine, dextropropoxyphene, diamorphine, fentanyl, alfentanil, sufentanil, hydrocodone, hydromorphone, ketobemidone, levomethadyl, levorphanol, meperidine, methadone, morphine, nalbuphine, opium, oxycodone, papaveretum, pentazocine, pethidine, phenoperidine, piritramide, dextropropoxyphene, remifentanil, sufentanil, tilidine, tramadol, codeine, dihydrocodeine, meptazinol, dezocine, eptazocine, flupirtine or a combination thereof.

The bone material may include or be mixed with a therapeutic agent including, but not limited to, an anti-inflammatory agent. An example of an anti-inflammatory agent includes, but is not limited to, clonidine, sulindac, sulfasalazine, naroxyn, diclofenac, indomethacin, ibuprofen, flurbiprofen, ketoprofen, aclofenac, aloxiprin, aproxen, aspirin, diflunisal, fenoprofen, mefenamic acid, naproxen, phenylbutazone, piroxicam, meloxicam, salicylamide, salicylic acid, desoxysulindac, tenoxicam, ketoralac, clonidine, flufenisal, salsalate, triethanolamine salicylate, aminopyrine, antipyrine, oxyphenbutazone, apazone, cintazone, flufenamic acid, clonixeril, clonixin, meclofenamic acid, flunixin, colchicine, demecolcine, allopurinol, oxypurinol, benzydamine hydrochloride, dimefadane, indoxole, intrazole, mimbane hydrochloride, paranylene hydrochloride, tetrydamine, benzindopyrine hydrochloride, fluprofen, ibufenac, naproxol, fenbufen, cinchophen, diflumidone sodium, fenamole, flutiazin, metazamide, letimide hydrochloride, nexeridine hydrochloride, octazamide, molinazole, neocinchophen, nimazole, proxazole citrate, tesicam, tesimide, tolmetin, triflumidate, fenamates (mefenamic acid, meclofenamic acid), nabumetone, celecoxib, etodolac, nimesulide, apazone, gold, tepoxalin; dithiocarbamate, or a combination thereof.

Anti-inflammatory agents also include steroids, such as for example, 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, dexamethasone 21-acetate, dexamethasone 21-phosphate di-Na salt, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide or a combination thereof.

The bone material may include or be mixed with a therapeutic agent including, but not limited to, a statin. Examples of a useful statin include, but are not limited to, atorvastatin, simvastatin, pravastatin, cerivastatin, mevastatin (see U.S. Pat. No. 3,883,140), velostatin (also called synvinolin; see U.S. Pat. Nos. 4,448,784 and 4,450,171 ), fluvastatin, lovastatin, rosuvastatin and fluindostatin (Sandoz XU-62-320), dalvastain (EP Appln. Publn. No. 738510 A2), eptastatin, pitavastatin, or pharmaceutically acceptable salts thereof or a combination thereof. In various embodiments, the statin may comprise mixtures of (+)R and (-)-S enantiomers of the statin. In various embodiments, the statin may comprise a 1:1 racemic mixture of the statin.

In some embodiments, the bone material can include an antimicrobial agent. In some embodiments, the antimicrobial agent can include one or more of triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, or combinations thereof.

Examples of antimicrobial agents include, by way of illustration and not limited to, acedapsone; acetosulfone sodium; alamecin; alexidine; amdinocillin; amdinocillin pivoxil; amicycline; amifloxacin; amifloxacin mesylate; amikacin; amikacin sulfate; aminosalicylic acid; aminosalicylate sodium; amoxicillin; amphomycin; ampicillin; ampicillin sodium; apalcillin sodium; apramycin; aspartocin; astromicin sulfate; avilamycin; avoparcin; azithromycin; azlocillin; azlocillin sodium; bacampicillin hydrochloride; bacitracin; bacitracin methylene disalicylate; bacitracin zinc; bambermycins; benzoylpas calcium; berythromycin; betamicin sulfate; biapenem; biniramycin; biphenamine hydrochloride; bispyrithione magsulfex; butikacin; butirosin sulfate; capreomycin sulfate; carbadox; carbenicillin disodium; carbenicillin indanyl sodium; carbenicillin phenyl sodium; carbenicillin potassium; carumonam sodium; cefaclor; cefadroxil; cefamandole; cefamandole nafate; cefamandole sodium; cefaparole; cefatrizine; cefazaflur sodium; cefazolin; cefazolin sodium; cefbuperazone; cefdinir; cefepime; cefepime hydrochloride; cefetecol; cefixime; cefmenoxime hydrochloride; cefmetazole; cefmetazole sodium; cefonicid monosodium; cefonicid sodium; cefoperazone sodium; ceforanide; cefotaxime sodium; cefotetan; cefotetan disodium; cefotiam hydrochloride; cefoxitin; cefoxitin sodium; cefpimizole; cefpimizole sodium; cefpiramide; cefpiramide sodium; cefpirome sulfate; cefpodoxime proxetil; cefprozil; cefroxadine; cefsulodin sodium; ceftazidime; ceftibuten; ceftizoxime sodium; ceftriaxone sodium; cefuroxime; cefuroxime axetil; cefuroxime pivoxetil; cefuroxime sodium; cephacetrile sodium; cephalexin; cephalexin hydrochloride; cephaloglycin; cephaloridine; cephalothin sodium; cephapirin sodium; cephradine; cetocycline hydrochloride; cetophenicol; chloramphenicol; chloramphenicol palmitate; chloramphenicol pantothenate complex; chloramphenicol sodium succinate; chlorhexidine phosphanilate; chloroxylenol; chlortetracycline bisulfate; chlortetracycline hydrochloride; cinoxacin; ciprofloxacin; ciprofloxacin hydrochloride; cirolemycin; clarithromycin; clinafloxacin hydrochloride; clindamycin; clindamycin hydrochloride; clindamycin palmitate hydrochloride; clindamycin phosphate; clofazimine; cloxacillin benzathine; cloxacillin sodium; chlorhexidine, cloxyquin; colistimethate sodium; colistin sulfate; coumermycin; coumermycin sodium; cyclacillin; cycloserine; dalfopristin; dapsone; daptomycin; demeclocycline; demeclocycline hydrochloride; demecycline; denofungin; diaveridine; dicloxacillin; dicloxacillin sodium; dihydrostreptomycin sulfate; dipyrithione; dirithromycin; doxycycline; doxycycline calcium; doxycycline fosfatex; doxycycline hyclate; droxacin sodium; enoxacin; epicillin; epitetracycline hydrochloride; erythromycin; erythromycin acistrate; erythromycin estolate; erythromycin ethylsuccinate; erythromycin gluceptate; erythromycin lactobionate; erythromycin propionate; erythromycin stearate; ethambutol hydrochloride; ethionamide; fleroxacin; floxacillin; fludalanine; flumequine; fosfomycin; fosfomycin tromethamine; fumoxicillin; furazolium chloride; furazolium tartrate; fusidate sodium; fusidic acid; ganciclovir and ganciclovir sodium; gentamicin sulfate; gloximonam; gramicidin; haloprogin; hetacillin; hetacillin potassium; hexedine; ibafloxacin; imipenem; isoconazole; isepamicin; isoniazid; josamycin; kanamycin sulfate; kitasamycin; levofuraltadone; levopropylcillin potassium; lexithromycin; lincomycin; lincomycin hydrochloride; lomefloxacin; lomefloxacin hydrochloride; lomefloxacin mesylate; loracarbef; mafenide; meclocycline; meclocycline sulfosalicylate; megalomicin potassium phosphate; mequidox; meropenem; methacycline; methacycline hydrochloride; methenamine; methenamine hippurate; methenamine mandelate; methicillin sodium; metioprim; metronidazole hydrochloride; metronidazole phosphate; mezlocillin; mezlocillin sodium; minocycline; minocycline hydrochloride; mirincamycin hydrochloride; monensin; monensin sodiumr; nafcillin sodium; nalidixate sodium; nalidixic acid; natainycin; nebramycin; neomycin palmitate; neomycin sulfate; neomycin undecylenate; netilmicin sulfate; neutramycin; nifuiradene; nifuraldezone; nifuratel; nifuratrone; nifurdazil; nifurimide; nifiupirinol; nifurquinazol; nifurthiazole; nitrocycline; nitrofurantoin; nitromide; norfloxacin; novobiocin sodium; ofloxacin; onnetoprim; oxacillin and oxacillin sodium; oximonam; oximonam sodium; oxolinic acid; oxytetracycline; oxytetracycline calcium; oxytetracycline hydrochloride; paldimycin; parachlorophenol; paulomycin; pefloxacin; pefloxacin mesylate; penamecillin; penicillins such as penicillin g benzathine, penicillin g potassium, penicillin g procaine, penicillin g sodium, penicillin v, penicillin v benzathine, penicillin v hydrabamine, and penicillin v potassium; pentizidone sodium; phenyl aminosalicylate; piperacillin sodium; pirbenicillin sodium; piridicillin sodium; pirlimycin hydrochloride; pivampicillin hydrochloride; pivampicillin pamoate; pivampicillin probenate; polymyxin b sulfate; porfiromycin; propikacin; pyrazinamide; pyrithione zinc; quindecamine acetate; quinupristin; racephenicol; ramoplanin; ranimycin; relomycin; repromicin; rifabutin; rifametane; rifamexil; rifamide; rifampin; rifapentine; rifaximin; rolitetracycline; rolitetracycline nitrate; rosaramicin; rosaramicin butyrate; rosaramicin propionate; rosaramicin sodium phosphate; rosaramicin stearate; rosoxacin; roxarsone; roxithromycin; sancycline; sanfetrinem sodium; sarmoxicillin; sarpicillin; scopafungin; sisomicin; sisomicin sulfate; sparfloxacin; spectinomycin hydrochloride; spiramycin; stallimycin hydrochloride; steffimycin; streptomycin sulfate; streptonicozid; sulfabenz; sulfabenzamide; sulfacetamide; sulfacetamide sodium; sulfacytine; sulfadiazine; sulfadiazine sodium; sulfadoxine; sulfalene; sulfamerazine; sulfameter; sulfamethazine; sulfamethizole; sulfamethoxazole; sulfamonomethoxine; sulfamoxole; sulfanilate zinc; sulfanitran; sulfasalazine; sulfasomizole; sulfathiazole; sulfazamet; sulfisoxazole; sulfisoxazole acetyl; sulfisboxazole diolamine; sulfomyxin; sulopenem; sultamricillin; suncillin sodium; talampicillin hydrochloride; teicoplanin; temafloxacin hydrochloride; temocillin; tetracycline; tetracycline hydrochloride; tetracycline phosphate complex; tetroxoprim; thiamphenicol; thiphencillin potassium; ticarcillin cresyl sodium; ticarcillin disodium; ticarcillin monosodium; ticlatone; tiodonium chloride; tobramycin; tobramycin sulfate; tosufloxacin; trimethoprim; trimethoprim sulfate; trisulfapyrimidines; troleandomycin; trospectomycin sulfate; tyrothricin; vancomycin; vancomycin hydrochloride; virginiamycin; zorbamycin; or combinations thereof.

The antimicrobial agent in the bone material can be an antiviral agent that can be mixed with the bone material. Antiviral agents can include, but are not limited to, vidarabine, acyclovir, famciclovir, valacyclovir, gancyclovir, valganciclovir, nucleoside-analog reverse transcriptase inhibitors (such as AZT (zidovudine), ddI (didanosine), ddC (zalcitabine), d4T (stavudine), and 3TC (lamivudine)), nevirapine, delavirdine, protease inhibitors (such as, saquinavir, ritonavir, indinavir, and nelfinavir), ribavirin, amantadine, rimantadine, neuraminidase inhibitors (such as zanamivir and oseltamivir), pleconaril, cidofovir, foscarnet, and/or interferons.

### Container for Mixing and/or Dispensing Bone Material

According to the invention as defined in claim 1, there is a container for mixing bone material provided, the container comprising, consisting essentially of or consisting of an interior surface configured for receiving bone material thereon and an exterior surface having a distal end and a proximal end, the distal end comprising a locking member configured to enclose the bone material onto the interior surface of the container, the proximal end of the exterior surface having a port configured to receive fluid to mix with the bone material on the interior surface of the container or withdraw bone material mixed with fluid on the interior surface from the container. The container can be provided preloaded with dry bone material by the manufacturer or it can be loaded during the surgical procedure.

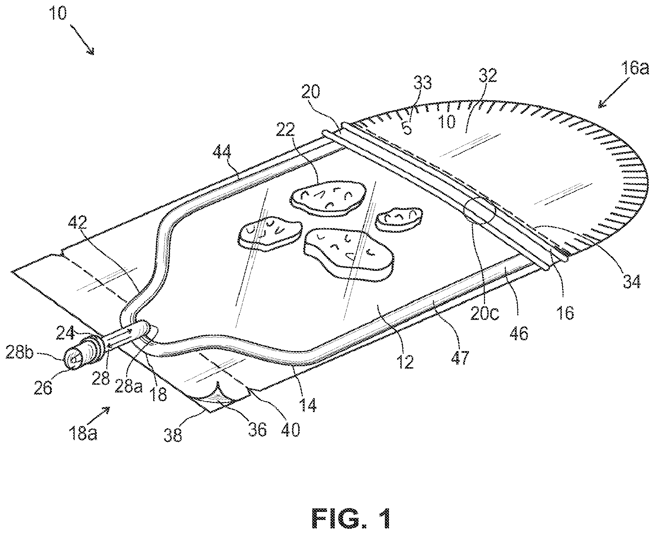

Referring to FIGS. 1-7, a container 10 is provided for mixing and dispensing bone material 22 (e.g., bone graft). Container 10 has an interior surface 12 configured for receiving bone material 12 onto this interior surface, an exterior surface 14 having a distal end 16 and a proximal end 18. At distal end 16 of the exterior surface there is a locking member 20 and at proximal end 18 of exterior surface 14 there is a port 26.

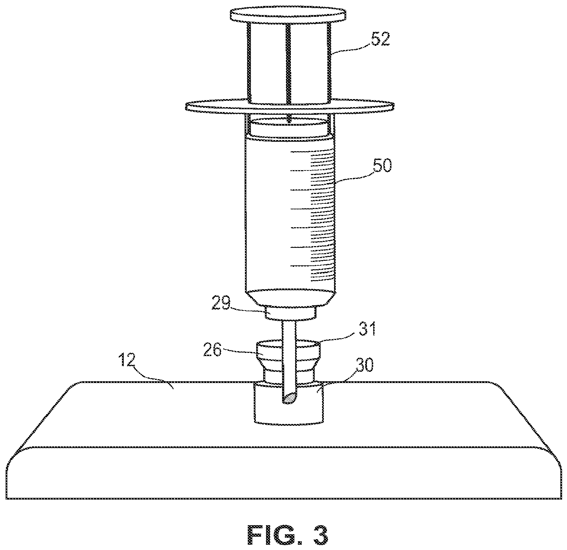

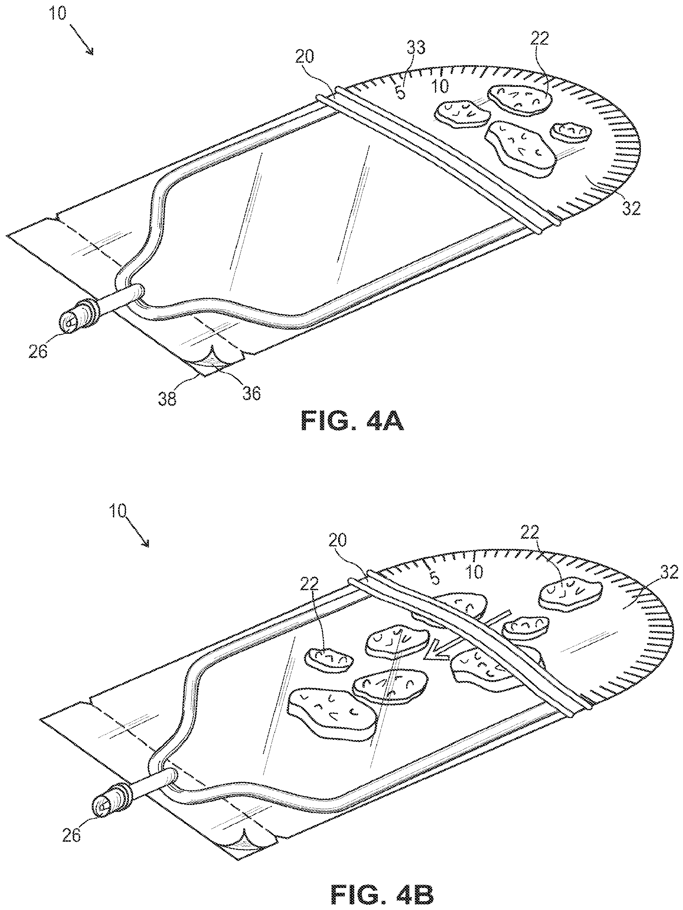

Port 26, in many aspects, is configured to receive fluid 24 onto or out of interior surface 12 of container 10 for mixing bone material 22 and/or for dispensing the bone material mixed with fluid 23. Port 26 contains channel 28 which has a proximal end 28a and distal end 28b. In some embodiments, port 26 and/or channel 28 contains an inside diaphragm 30, an outside diaphragm 31 or a fitting 29 at its proximal end, for example, a luer fitting configured to engage a syringe 50 having a plunger 52 and/or another fluid providing device as illustrated in FIG. 3. In other embodiments, port 26 is removable from container 10 as illustrated in FIG. 4A. At its distal end, channel 28 is connected to neck 42 located at the proximal end 18 of exterior surface 14. In some aspects, the proximal end 18a of container 10 comprises neck 42.

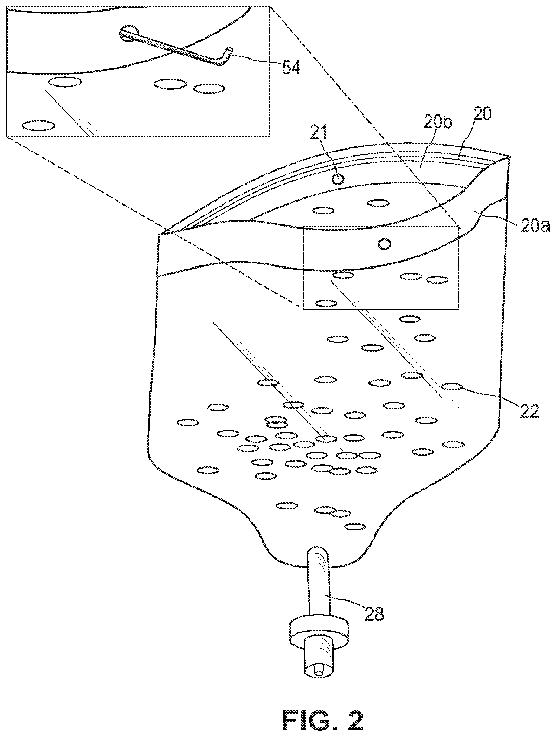

Locking member 20 encloses bone material 22 onto interior surface 12 of container 10 when in a closed position and allows bone material 22 onto interior surface 12 when in an open position as illustrated in FIGS. 2 and 4B. Locking member 20 comprises, consists essentially of or consists of a first locking member 20a disposed on exterior surface 14, a second corresponding locking member 20b disposed on interior surface 12 and, in some aspects, a sliding member 20c to allow a seal between the exterior surface and the interior surface of container 10 when the respective locking members are engaged to prevent loss of dry bone material and/or when mixed with fluid present or later added to the container. Locking member 20 is resealable. In the embodiment illustrated in FIG. 2, locking member 20 contains an aperture 21 centrally disposed on first locking member 20a and/or second corresponding locking member 20b. Aperture 21 can be used to hang container 10 from a hanging hook 54, for example.

According to the invention, exterior surface 14 comprises a staging surface 32 disposed adjacent locking member 20 for receiving, staging, and/or measuring the bone material for entry onto the interior surface 12 when locking member 20 is in an open position. The staging surface may comprise measuring indicia 33 and a tear line 34 configured for tearing the staging surface from container 10.

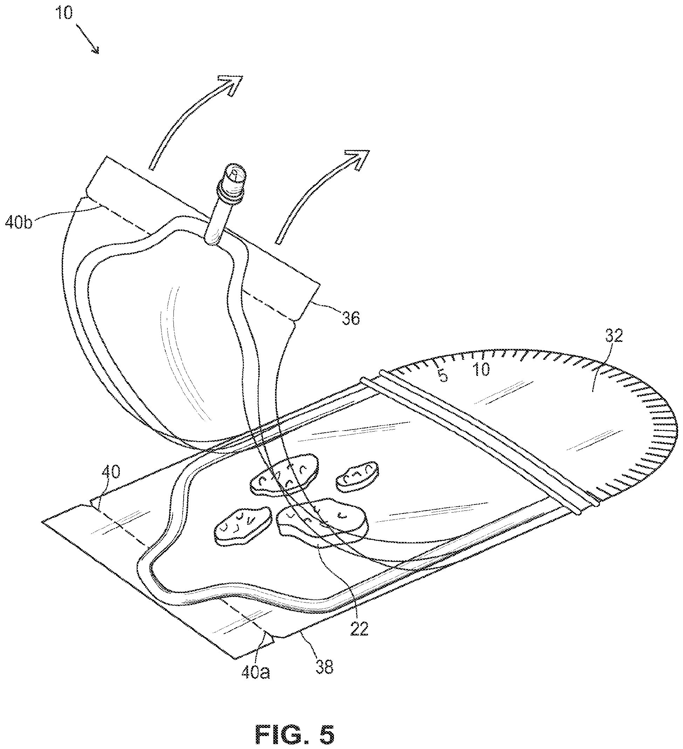

In many aspects, container 10 comprises an upper layer 36 which includes exterior surface 14 and a lower layer 38 which includes interior surface 12. Upper layer 36 can be peelable incrementally from lower layer 38 in a direction towards the distal end 16 of container 10 to allow access to bone material 22 and/or fluid 24 on the interior surface 12 of container 10. In some aspects, upper layer 36 and lower layer 38 have sidewalls 44 and 46 that form a perimeter seal 47 about container 10. In other aspects, as illustrated in FIG. 5, upper layer 36 and lower layer 38 have tear lines 40a and 40b, both adjacent port 26 and together forming tear line 40. In some embodiments, by tearing along tear lines 40a and/or 40b, the port can be permanently removed to form opening 48 in container 10 for dispensing bone material and/or fluid from the opening.

The container comprises non-porous, non-biodegradable material, transparent and/or impermeable material. In some aspects, the container can be made from polymers such as linear high-density polyethene (HDPE) branched low-density polyethene (LDPE) ultra-high molecular weight polyethylene(UHMWPE), polyethylene terephthalate (PET), polyvinyl chloride (V or vinyl or PVC), polypropylene (PP), polystyrene(PS). Laminated plastics can also be used as materials for the interior and/or exterior surfaces of the container described in this application. Laminated plastics are a special form of polymer-matrix composite consisting of layers of reinforcing materials that have been impregnated with thermosetting resins, bonded together, and cured under heat and pressure. Nonwoven materials made from high density polyethylene fibers, for example, Tyvek^{®} is also useful. Electrospun material, made by needling material to create a felt, point-bonded, spun-bonded or a combination thereof is useful in preparing the interior and/or exterior surfaces of the container. In various embodiments, the container comprises, consists essentially of or consists of a nonwoven material or fabric which in some aspects, can be in a sheet form. In various aspects, a nonwoven material is a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin. In various embodiments, the material used in making the interior and exterior surface of the container can be made of the same or different material. In some embodiments, one surface can be made of a polymer while the other can be prepared of metal foil. In other embodiments, the material of the container can be sufficiently porous so that the material enclosed therein can be freeze-dried and/or sterilized.

The container is flexible. In some embodiments, the container has sufficient flexibility such that it can be kneadable by hand. In some embodiments, the exterior surface and the interior surface of the container are kneadable. In some embodiments, the container can be made from a material having a modulus of elasticity from about 1×10² to about 6×10⁵ dynes/cm², or 2×10⁴ to about 5×10⁵ dynes/cm², or 5×10⁴ to about 5×10⁵ dynes/cm². In some embodiments, the container can be made from polyethylene terephthalate (PET) and has a modulus of elasticity of about 3.15×10⁸ dynes/cm². In some embodiments, the container is flexible when kneaded by hand. In some embodiments, the container is able to resist stretching.

In some embodiments, components of the container can be fabricated from biologically acceptable materials suitable for medical applications, including metals, synthetic polymers, ceramics and/or their composites. For example, the components of the container, individually or collectively, can be fabricated from other materials such as stainless steel alloys, commercially pure titanium, titanium alloys, Grade 5 titanium, super-elastic titanium alloys, cobalt-chrome alloys, stainless steel alloys, superelastic metallic alloys (e.g., Nitinol, super elasto-plastic metals, such as GUM METAL^{®} manufactured by Toyota Material Incorporated of Japan), ceramics and composites thereof such as calcium phosphate (e.g., SKELITETM manufactured by Biologix Inc.), thermoplastics such as polyaryletherketone (PAEK) including polyetheretherketone (PEEK), polyetherketoneketone (PEKK) and polyetherketone (PEK), carbon-PEEK composites, PEEK-BaSO4 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, or any combination thereof.

In some embodiments, the container 10 can be made of a memory shape polymer and/or alloy to allow the container 10 to move from an unfolded configuration to a folded or rolled configuration without the need for a locking mechanism. In some embodiments, a memory shape material may be used in the interior and exterior surfaces of the container 10, such as a memory shape polymer or alloys. Memory shape polymers include, but are not limited to polyethers, polyacrylates, polyamides, polysiloxanes, polyurethanes, polyethers amides, polyurethane/ureas, polyether esters, polynorborene, cross-linked polymers such as cross-linked polyethylene and cross-linked poly(cyclooctene), inorganic-organic hybrid polymers, and copolymers such as urethane/butadiene copolymers, styrene-butadiene copolymers. Memory shape alloys include, but are not limited to TiNi, CuZnAl, and FeNiAl alloys. In some embodiments, the container 10 can be fabricated by injection molding of plastic materials comprising rigid, surgical grade plastic and/or metal materials.

The container can also be made by additive manufacturing methods (e.g., 3D printing). In some embodiments, the components of the container, such as the interior and exterior surfaces of the container can be made from an impermeable yarn that is monofilament or multifilament, and the yarn can be knitted, woven, non-woven shape memory, felted, point-bonded, additive manufactured, such as 3-D printed or a combination thereof.

In some embodiments, fluid 24 can be glycerol, blood, bone marrow aspirate, mesenchymal stem cells, sterile water, dextrose, other sugars including but not limited to sucrose, fructose, glucose, lactated ringer's, polyols including, but not limited to, mannitol, xylitol, sorbitol, maltitol, lactitol, polysaccharides including, but not limited to, native or pre-gelatinized starch, maltodextrins, cyclodextrins, mineral compounds including, but not limited to, dicalcium or tricalcium phosphate, either dihydrate or anhydrous, cellulose derivatives including, but not limited to, microcrystalline cellulose, lactoses either monohydrates thereof or anhydrous, as well as their mixtures such as dicalcium phosphate dihydrate, mannitol, pre-gelatinized maize starch, microcrystalline cellulose and their mixtures, water and/or NaCl (saline). In some embodiments, the saline is 0.90% saline, 0.45% saline or phosphate buffered saline. In some embodiments, other fluids can be used for example, D5W (dextrose in 5% water), D5NS (dextrose in 5% water and normal saline) and D5W/½NS (D5W and ½ normal saline), lactated Ringer solution or the like. In other embodiments, the fluid comprises bone marrow aspirate, saline, sterile water, blood for injection, phosphate buffered saline, dextrose, Ringer's lactated solution, or a combination thereof.

FIGS. 4A to 7 are representative illustrations of container 10 in use. For example, FIG. 4A shows bone material 22 placed on staging surface 32 in the proximity of locking member 20. Locking member 20 can be opened via sliding member 20c to access interior surface 12 so that bone material 22 can be placed onto interior surface 12 for mixing with fluid 24 that can be added through port 26. FIG. 4B illustrates bone material 22 that has advanced onto the interior surface 12. Once inside container 10, locking member 20 can be closed and bone material 22 can be manually mixed with fluid 24. In various embodiments, container 10 comprises flexible sidewalls 44 and 46, which are configured to allow manual mixing of fluid 24 and bone material 22 on the interior surface 12 by pressure applied by hand. Fluid 24 can be added through port 26 which in some aspects, is configured to receive syringe 50 having a plunger 52 to deliver fluid to container 10 or to be used for precise dispensing of bone material from container 10 at a surgical site, for example.

In some embodiments, as illustrated in FIG. 5, container 10 includes an upper layer 36 having exterior surface 14 and a lower layer 38 having the interior surface 12. The bone material and/or fluid can be accessed by peeling gradually or completely upper layer 36 which includes exterior surface 14 in a direction towards the distal end of container 10.

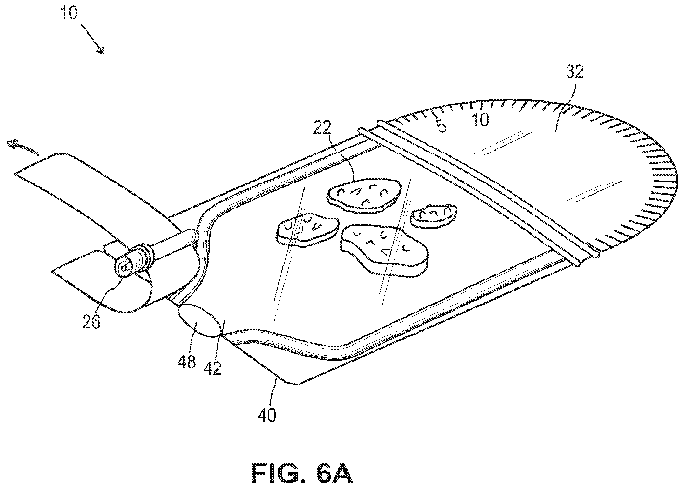

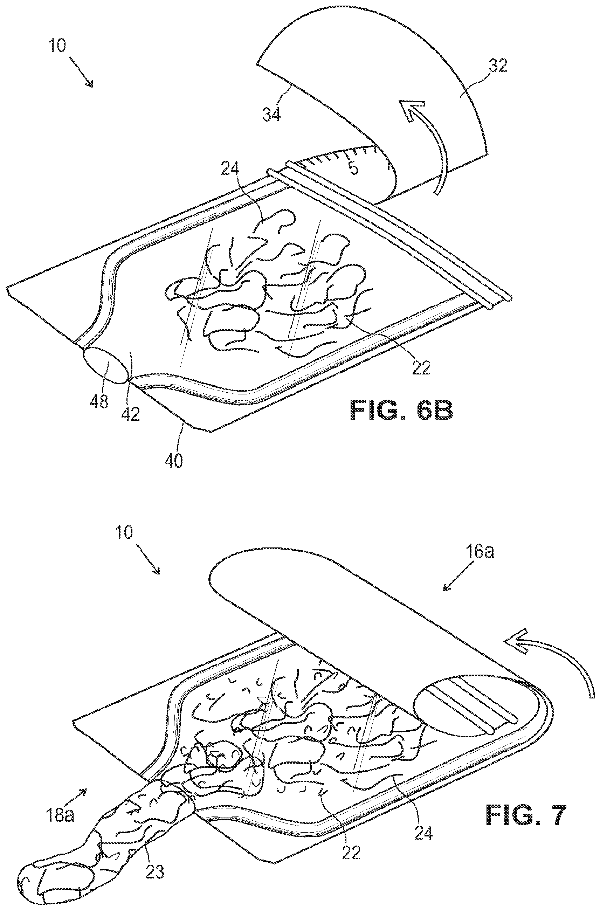

In other embodiments, as illustrated in FIGS. 6A and 6B, the bone material and/or fluid can be removed from container 10 by peeling off the upper layer and/or the lower layer at tear lines 40a and/or 40b disposed adjacent to port 26 so as to remove the port and form opening 48 disposed by neck 42. As also illustrated in FIG. 6B, staging surface 32 can also be removed by tearing along tear line 34. In various embodiments, as illustrated in FIG. 7, after mixing the bone material 22 with fluid 24, the mixed bone material and fluid 23 can be squeezed out of container 10 through opening 48 by rolling the distal end 16a of container 10 toward the proximal end 18a of container 10. The distal end of the container is configured to be rolled toward the proximal end of the container to dispense from the container bone material and/or fluid. In many embodiments, container 10 can be covered with other coverings, a second or even a third covering (not shown) that can be peeled off the container. Such additional coverings may be used to preserve the sterility of container 10 and/or prevent oxidation of material that may be present onto the interior surface of container 10. Nevertheless, in some embodiments, the container can be provided empty, without any material including bone material, which can be provided separately at a surgical site.

In some embodiments, the container is multifunctional and allows for, among other things, long-term safe storage and containment of bone material, easy and safe addition and/or removal of fluids to the bone material, easy visualization and manual mixing of bone material, easy addition of bone material via a resealable container and integrated staging surface (e.g., tongue-like landing pad/funnel/staging feature), precise dispensing via built in port, and/or bulk access via peelable locking surface. The container may include measuring features.

In some embodiments, the container allows storage, removal or addition of fluid and manipulation of bone material in a closed system, which can lead to less chance for bioburden contamination and/or drying-out of bone material prior to use.

A kit may be provided comprising the bone material container. The kit may include additional parts along with the bone material container including the bone material and other components to be used to administer the bone material (e.g., spatulas, wipes, needles, syringes, delivery cannulas, other mixing devices, etc.). Delivery cannulas could be designed to connect or interface with other delivery systems and/or could be designed to connect directly to the container allowing for precise dispensing of the bone graft via the built in port or fitting. The kit may include the bone material in a first compartment. The second compartment may include a vial holding the carrier fluid and any other instruments needed for the delivery. A third compartment may include gloves, drapes, wound dressings and other procedural supplies for maintaining sterility of the implanting process, as well as an instruction booklet, which may include a chart that shows how to administer the bone material after mixing it. A fourth compartment may include additional needles and/or sutures. Each tool may be separately packaged in a plastic pouch that is sterilized. A fifth compartment may include an agent for radiographic imaging. A cover of the kit may include illustrations of the relevant surgical procedure and a clear plastic cover may be placed over the compartments to maintain sterility.

In various embodiments, one or more components of the bone material container is sterilized by radiation in a terminal sterilization step in the final packaging. Terminal sterilization of a product provides greater assurance of sterility than from processes such as an aseptic process, which require individual product components to be sterilized separately and the final package assembled in a sterile environment.

In various embodiments, gamma radiation is used in the terminal sterilization step, which involves utilizing ionizing energy from gamma rays that penetrates deeply into the bone material container. Gamma rays are highly effective in killing microorganisms, they leave no residues, nor do they have sufficient energy to impart radioactivity to the container. Gamma rays can be employed when the container is in the package and gamma sterilization does not require high pressures or vacuum conditions, thus, package seals and other components are not stressed. In addition, gamma radiation eliminates the need for permeable packaging materials.

In various embodiments, electron beam (e-beam) radiation may be used to sterilize one or more components of the container for mixing bone material. E-beam radiation comprises a form of ionizing energy, which is generally characterized by low penetration and high-dose rates. E-beam irradiation is similar to gamma processing in that it alters various chemical and molecular bonds on contact, including the reproductive cells of microorganisms. Beams produced for e-beam sterilization are concentrated, highly-charged streams of electrons generated by the acceleration and conversion of electricity.

In various embodiments, gamma radiation, e-beam radiation, steam, or gas is used to presterilize the container prior to aseptic filling of the container with bone material in a clean and controlled environment resulting in a sterile finished device that did not require terminal sterilization of the finished packaged bone material.

Other methods may also be used to sterilize the container for mixing bone material including, but not limited to, gas sterilization such as, for example, with ethylene oxide or steam sterilization.

### Methods of Use

A method of mixing bone material 22 into container 10 is provided according to the invention as defined in claim 13. The method comprises providing a container for mixing bone material, the container comprising an interior surface for receiving bone material thereon and an exterior surface having a distal end and a proximal end, the distal end comprising a locking member configured to enclose the bone material onto the interior surface of the container, the proximal end having a port configured to receive fluid to mix with the bone material on the interior surface of the container or withdraw bone material mixed with fluid on the interior surface of the container; opening the locking member and adding the bone material onto the interior surface of the container; closing the locking member to enclose the bone material on the interior surface of the container; and adding fluid to the container by passing fluid through the port of the container. According to the invention, the container has a staging surface to facilitate and/or measure adding bone material to the container. In many embodiments, the bone material comprises ceramic, collagen, allograft bone, autograft bone, demineralized bone matrix fibers, demineralized bone powder, demineralized bone chips or a combination thereof.

The bone material can have excipients, such as for example, mannitol, alginate, sodium or potassium phosphate, citric acid, tartaric acid, gelatin, glycine, lactose, sucrose, maltose, glycerin, dextrose, dextran, trehalose, hetastarch, ascorbic acid, acetylcysteine, cysteine, sodium hydrogen sulfite, butyl-hydroxyanisole, butyl-hydroxytoluene or alpha-tocopherol acetate, and/or chelators.

In some embodiments, the bone material is relatively dry and also includes dry excipients which can be hydrated or mixed with a liquid at time of surgery. Some examples of dry excipients include, for example, starches, alginates, collagens, gelatins, chitosans, Xantham gums, cellulose, carboxymethylcellulose, polyethylene glycol, polyvinylpyrrolidone, talc, magnesium stearate, glyceryl behenate, stearic acid, and/or titanium dioxide.

The bone material can be pre-loaded into the container in a relatively dry state and can be then mixed with liquid material and optionally a therapeutic agent using the spatula when the locking mechanism is open, or by manually kneading the container when the locking mechanism is closed until the desired consistency of the bone material is achieved (e.g., putty, paste, etc.). The pre-loaded bone material can be relatively wet prior to mixing with liquid. The bone material can be mixed externally to the container with a suitable diluent and then loaded into the container through the locking mechanism. In some embodiments, the diluent includes dextrose, other sugars including but not limited to sucrose, fructose, glucose, lactated ringer's, polyols including but not limited to mannitol, xylitol, glycerol, sorbitol, maltitol, lactitol, polysaccharides including but not limited to native or pre-gelatinized starch, maltodextrins, cyclodextrins, mineral compounds including but not limited to dicalcium or tricalcium phosphate, either dihydrate or anhydrous, cellulose derivatives including but not limited to microcrystalline cellulose, lactoses either monohydrates thereof or anhydrous, as well as their mixtures such as dicalcium phosphate dihydrate, mannitol, pre-gelatinized maize starch, microcrystalline cellulose and their mixtures, water and/or NaCl (saline). In some embodiments, the saline is 0.90% saline or 0.45% saline. In some embodiments, other delivery vehicles can be used for example, D5W (dextrose in 5% water), D5NS (dextrose in 5% water and normal saline) and D5W/1/2NS (D5Wand ½ normal saline), blood, mesenchymal stem cells, or the like.

The method may comprise removing the staging surface from the container and/or removing the port from the container by removing an upper layer comprising the exterior surface and/or a lower layer comprising the interior surface. The method of mixing bone material may include peeling away the upper layer from the lower layer partially or completely in a direction towards the distal end of the container to allow access to any bone and/or fluid on the interior surface of the container. The method of mixing bone material may include rolling the distal end of the container toward the proximal end of the container to dispense bone material and/or fluid from the container. The method of mixing bone material may include removing the port by peeling the upper layer and/or the lower layer along tear lines adjacent to the port.

The container for mixing bone material can be used to treat a variety of conditions including osteoporosis, bone fracture repair or healing, dental procedures for which increased bone formation in the jaw is of clinical benefit, repair of craniofacial bone defects induced by trauma or congenital defects such as cleft palate/lip, and a number of other musculoskeletal disorders where native bone growth is inadequate, which will be evident to those of ordinary skill in the art. The bone material can be administered to treat open fractures and fractures at high risk of non-union, and in subjects with spinal disorders, including subjects in need of spine fusion (e.g., anterior lumbar interbody fusion, posterior lumbar spinal fusion, and cervical spine fusion) or subjects having degenerative disc disease or arthritis affecting the lumbar and cervical spine.

Although the invention has been described with reference to embodiments, persons skilled in the art will recognize that changes may be made in form and detail without departing from the scope of the invention, which is defined by the claims.

## Claims

1. A container (10) for mixing bone material (22), the container (10) comprising an interior surface (12) configured for receiving bone material (22) thereon and an exterior surface (14) having a distal end (16) and a proximal end (18), the distal end (16) comprising a locking member (20) configured to enclose the bone material (22) onto the interior surface (12) of the container (10), the proximal end (18) of the exterior surface (14) having a port (26) configured to receive fluid (24) to mix with the bone material (22) on the interior surface (12) of the container (10) or withdraw bone material (22) mixed with fluid (24) on the interior surface (12) from the container (10);
**characterized in that** the exterior surface (14) further comprises a staging surface (32) adjacent to the locking member (20) configured to receive and measure the bone material (22) for entry of the bone material (22) onto the interior surface (12) when the locking member (20) is in an open position.

2. The container (10) of claim 1, wherein the port (26) comprises a channel (28) to allow fluid (24) onto or out of the interior surface (12) of the container (10), wherein, optionally, the fluid (24) comprises bone marrow aspirate, saline, sterile water, blood for injection, phosphate buffered saline, dextrose, Ringer's lactated solution, or a combination thereof.

3. The container (10) of claim 2, wherein (i) the port (26) and/or channel (28) has a diaphragm (30) to allow fluid (24) onto or out of the interior surface (12) of the container (10); (ii) the port (26) has a fitting configured to engage a syringe (50); or (iii) the port (26) and/or channel (28) is removable from the container (10).

4. The container (10) of one of claims 1-3, wherein the locking member (20) (i) encloses the bone material (22) onto the interior surface (12) of the container (10) when in a closed position and allows the bone material (22) entry onto the interior surface (12) of the container (10) when in an open position; (ii) comprises a first locking member (20) disposed on the exterior surface (14) and a corresponding locking member (20) disposed on the interior surface (12) to allow a seal between the exterior surface (14) and the interior surface (12) when the respective locking members (20) are engaged to prevent loss of bone material (22) or fluid (24) mixed with the bone material (22) from the container (10); or (iii) is resealable.

5. The container (10) of one of claims 1-4, wherein the staging surface (32) comprises a tear line (34) configured for removing the staging surface (32) from the container (10).

6. The container (10) of one of claims 1-5, comprising an upper layer (36) having the exterior surface (14) and a lower layer (38) having the interior surface (12).

7. The container (10) of claim 6, wherein (i) the upper layer (36) is peelable away from the lower layer (38) and in a direction towards the distal end (16) of the container (10) to allow access to any bone material (22) and/or fluid (24) on the interior surface (12) of the container (10); (ii) the upper layer (36) and the lower layer (38) have sidewalls (44) that form a perimeter seal (47) about the container; or (iii) the upper layer (36) and the lower layer (38) have tear lines (34) adjacent to the port (26), the tear lines (34) configured to allow permanent removal of the port (26) so as to form an opening (48) in the container (10) for dispensing bone material (22) and/or fluid (24) from the opening (48).

8. The container (10) of one of claims 1-7, wherein the container (10) comprises non-porous material, transparent and/or impermeable material.

9. The container (10) of one of claims 1-8, wherein the bone material (22) comprises ceramic, collagen, allograft bone, autograft bone, demineralized bone matrix fibers, demineralized bone powder, demineralized bone chips or a combination thereof.

10. The container (10) of one of claims 1-9, wherein (i) the proximal end (18) of the container (10) comprises a neck (42); or (ii) the distal end (16) of the container (10) is configured to be rolled and/or squeezed toward the proximal end (18) of the container (10) to dispense bone material (22) and/or fluid (24) from the container (10).

11. The container (10) of one of claims 1-10, wherein the container (10) comprises flexible sidewalls (44) configured to allow manual mixing of fluid (24) and the bone material (22) on the interior surface (12) by pressure applied by hand.

12. The container (10) of one of claims 1-11, wherein the port (26) is configured to receive a syringe (50) for addition of fluid (24) to the container (10) or precise dispensing of bone material (22) from the container (10).

13. A method of mixing bone material (22), the method comprises providing a container (10) for mixing bone material (22), the container (10) comprising an interior surface (12) for receiving bone material (22) thereon and an exterior surface (14) having a distal end (16) and a proximal end (18), the distal end (16) comprising a locking member (20) configured to enclose the bone material (22) onto the interior surface (12) of the container (10), the proximal end (18) having a port (26) configured to receive fluid (24) to mix with the bone material (22) on the interior surface (12) of the container (10) or withdraw bone material (22) mixed with fluid (24) on the interior surface (12) of the container (10); opening the locking member (20) and adding the bone material (22) onto the interior surface (12) of the container (10); closing the locking member (20) to enclose the bone material (22) on the interior surface (12) of the container (10); and adding fluid (24) to the container (10) by passing fluid (24) through the port (26) of the container (10), wherein, optionally, the container (10) is sterile, **characterized in that** the container (10) has a staging surface (32) adjacent to the locking member (20) configured to receive and measure bone material (22) added to the container (10) for entry of the bone material (22) onto the interior surface (12) when the locking member (20) is in an open position.

14. The method of claim 13, wherein the bone material (22) comprises ceramic, collagen, allograft bone, autograft bone, demineralized bone matrix fibers, demineralized bone powder, demineralized bone chips or a combination thereof.

15. The method of claim 13 or 14, wherein the fluid (24) comprises bone marrow aspirate, saline, sterile water, blood for injection, phosphate buffered saline, dextrose, Ringer's lactated solution, or a combination thereof.

16. The method of one of claims 13-15, wherein the container (10) comprises non-porous material, transparent and/or impermeable material.

## Patentansprüche

1. Behälter (10) zum Mischen von Knochenmaterial (22), wobei der Behälter (10) eine Innenoberfläche (12), die zum Aufnehmen von Knochenmaterial (22) darauf konfiguriert ist, und eine Außenoberfläche (14) mit einem distalen Ende (16) und einem proximalen Ende (18) umfasst, wobei das distale Ende (16) ein Verriegelungselement (20) umfasst, das konfiguriert ist, um das Knochenmaterial (22) auf der Innenoberfläche (12) des Behälters (10) zu umschließen, wobei das proximale Ende (18) der Außenoberfläche (14) einen Anschluss (26) aufweist, der dazu konfiguriert ist, um Fluid (24) aufzunehmen, zum Mischen mit dem Knochenmaterial (22) auf der Innenoberfläche (12) des Behälters (10) oder zum Entnehmen von Knochenmaterial (22), das mit Fluid (24) auf der Innenoberfläche (12) des Behälters (10) gemischt ist;
**dadurch gekennzeichnet, dass** die Außenoberfläche (14) ferner eine Bereitstellungsoberfläche (32) angrenzend an das Verriegelungselement (20) umfasst, die konfiguriert ist, um das Knochenmaterial (22) zum Eintritt des Knochenmaterials (22) auf die Innenoberfläche (12) aufzunehmen und zu messen, wenn sich das Verriegelungselement (20) in einer offenen Position befindet.

2. Behälter (10) nach Anspruch 1, wobei der Anschluss (26) einen Kanal (28) umfasst, um Fluid (24) auf die oder aus der Innenoberfläche (12) des Behälters (10) zu lassen, wobei optional das Fluid (24) Knochenmarkaspirat, Kochsalzlösung, steriles Wasser, Blut für Injektion, phosphatgepufferte Kochsalzlösung, Dextrose, Ringer-Laktatlösung oder eine Kombination davon umfasst.

3. Behälter (10) nach Anspruch 2, wobei (i) der Anschluss (26) und/oder der Kanal (28) eine Membran (30) aufweist/aufweisen, um Fluid (24) auf die oder aus der Innenoberfläche (12) des Behälters (10) zu lassen; (ii) der Anschluss (26) ein Anschlussstück aufweist, das zum Eingriff mit einer Spritze (50) konfiguriert ist; oder (iii) der Anschluss (26) und/oder der Kanal (28) aus dem Behälter (10) entfernbar ist/sind.

4. Behälter (10) nach einem der Ansprüche 1-3, wobei das Verriegelungselement (20) (i), wenn es in einer geschlossenen Position ist, das Knochenmaterial (22) auf der Innenoberfläche (12) des Behälters (10) umschließt, und, wenn es in einer offenen Position ist, das Knochenmaterial (22) auf die Innenoberfläche (12) des Behälters (10) eintreten lässt; (ii) ein erstes Verriegelungselement (20), das auf der Außenoberfläche (14) angeordnet ist, und ein entsprechendes Verriegelungselement (20), das auf der Innenoberfläche (12) angeordnet ist, umfasst, um eine Abdichtung zwischen der Außenoberfläche (14) und der Innenoberfläche (12) zu ermöglichen, wenn die jeweiligen Verriegelungselemente (20) in Eingriff stehen, um einen Verlust von Knochenmaterial (22) oder Fluid (24), das mit dem Knochenmaterial (22) gemischt ist, aus dem Behälter (10) zu verhindern; oder (iii) wiederverschließbar ist.

5. Behälter (10) nach einem der Ansprüche 1-4, wobei die Bereitstellungsoberfläche (32) eine Reißlinie (34) umfasst, die zum Entfernen der Bereitstellungsoberfläche (32) von dem Behälter (10) konfiguriert ist.

6. Behälter (10) nach einem der Ansprüche 1-5, umfassend eine obere Schicht (36), die die Außenoberfläche (14) aufweist, und eine untere Schicht (38), die die Innenoberfläche (12) aufweist.

7. Behälter (10) nach Anspruch 6, wobei (i) die obere Schicht (36) von der unteren Schicht (38) weg und in einer Richtung zu dem distalen Ende (16) des Behälters (10) abziehbar ist, um Zugang zu einem beliebigen Knochenmaterial (22) und/oder Fluid (24) auf der Innenoberfläche (12) des Behälters (10) zu ermöglichen; (ii) die obere Schicht (36) und die untere Schicht (38) Seitenwände (44) aufweisen, die eine Umfangsdichtung (47) um den Behälter herum bilden; oder (iii) die obere Schicht (36) und die untere Schicht (38) Reißlinien (34) angrenzend an den Anschluss (26) aufweisen, wobei die Reißlinien (34) konfiguriert sind, um eine permanente Entfernung des Anschlusses (26) zu ermöglichen, um eine Öffnung (48) in dem Behälter (10) zum Abgeben von Knochenmaterial (22) und/oder Fluid (24) aus der Öffnung (48) zu bilden.

8. Behälter (10) nach einem der Ansprüche 1-7, wobei der Behälter (10) nichtporöses Material, transparentes und/oder undurchlässiges Material umfasst.

9. Behälter (10) nach einem der Ansprüche 1-8, wobei das Knochenmaterial (22) Keramik, Kollagen, Allotransplantatknochen, Autotransplantatknochen, demineralisierte Knochenmatrixfasern, demineralisiertes Knochenpulver, demineralisierte Knochenspäne oder eine Kombination davon umfasst.

10. Behälter (10) nach einem der Ansprüche 1-9, wobei (i) das proximale Ende (18) des Behälters (10) einen Hals (42) umfasst; oder (ii) das distale Ende (16) des Behälters (10) konfiguriert ist, um zum proximalen Ende (18) des Behälters (10) gerollt und/oder zusammengedrückt zu werden, um Knochenmaterial (22) und/oder Fluid (24) aus dem Behälter (10) abzugeben.

11. Behälter (10) nach einem der Ansprüche 1-10, wobei der Behälter (10) flexible Seitenwände (44) umfasst, die konfiguriert sind, um ein manuelles Mischen von Fluid (24) und dem Knochenmaterial (22) auf der Innenoberfläche (12) durch Druck, der von Hand ausgeübt wird, zu ermöglichen.

12. Behälter (10) nach einem der Ansprüche 1-11, wobei der Anschluss (26) konfiguriert ist, um eine Spritze (50) zum Hinzufügen von Fluid (24) zu dem Behälter (10) oder zum präzisen Abgeben von Knochenmaterial (22) aus dem Behälter (10) aufzunehmen.

13. Verfahren zum Mischen von Knochenmaterial (22), wobei das Verfahren umfasst: Bereitstellen eines Behälters (10) zum Mischen von Knochenmaterial (22), wobei der Behälter (10) eine Innenoberfläche (12) zum Aufnehmen von Knochenmaterial (22) darauf und eine Außenoberfläche (14) mit einem distalen Ende (16) und einem proximalen Ende (18) umfasst, wobei das distale Ende (16) ein Verriegelungselement (20) umfasst, das konfiguriert ist, um das Knochenmaterial (22) auf der Innenoberfläche (12) des Behälters (10) zu umschließen, wobei das proximale Ende (18) einen Anschluss (26) aufweist, der konfiguriert ist, um Fluid (24) aufzunehmen, zum Mischen mit dem Knochenmaterial (22) auf der Innenoberfläche (12) des Behälters (10) oder zum Entnehmen von Knochenmaterial (22), das mit Fluid (24) auf der Innenoberfläche (12) des Behälters (10) gemischt ist; Öffnen des Verriegelungselements (20) und Hinzufügen des Knochenmaterials (22) auf die Innenoberfläche (12) des Behälters (10); Schließen des Verriegelungselements (20), um das Knochenmaterial (22) auf der Innenoberfläche (12) des Behälters (10) zu umschließen; und Hinzufügen von Fluid (24) zu dem Behälter (10) durch Leiten von Fluid (24) durch den Anschluss (26) des Behälters (10), wobei optional der Behälter (10) steril ist,
**dadurch gekennzeichnet, dass** der Behälter (10) eine Bereitstellungsoberfläche (32) angrenzend an das Verriegelungselement (20) aufweist, die konfiguriert ist, um Knochenmaterial (22) aufzunehmen und zu messen, das dem Behälter (10) zum Eintritt des Knochenmaterials (22) auf die Innenoberfläche (12) hinzugefügt wird, wenn sich das Verriegelungselement (20) in einer offenen Position befindet.

14. Verfahren nach Anspruch 13, wobei das Knochenmaterial (22) Keramik, Kollagen, Allotransplantatknochen, Autotransplantatknochen, demineralisierte Knochenmatrixfasern, demineralisiertes Knochenpulver, demineralisierte Knochenspäne oder eine Kombination davon umfasst.

15. Verfahren nach Anspruch 13 oder 14, wobei das Fluid (24) Knochenmarkabsaugung, Kochsalzlösung, steriles Wasser, Blut für Injektion, phosphatgepufferte Kochsalzlösung, Dextrose, Ringer-Laktatlösung oder eine Kombination davon umfasst.

16. Verfahren nach einem der Ansprüche 13-15, wobei der Behälter (10) nichtporöses Material, transparentes und/oder undurchlässiges Material umfasst.

## Revendications

1. Récipient (10) permettant de mélanger un matériau osseux (22), le récipient (10) comprenant une surface intérieure (12) conçue pour recevoir un matériau osseux (22) sur celui-ci et une surface extérieure (14) ayant une extrémité distale (16) et une extrémité proximale (18), l'extrémité distale (16) comprenant un élément de verrouillage (20) conçu pour enfermer le matériau osseux (22) sur la surface intérieure (12) du récipient (10), l'extrémité proximale (18) de la surface extérieure (14) ayant un orifice (26) conçu pour recevoir un fluide (24) à mélanger avec le matériau osseux (22) sur la surface intérieure (12) du récipient (10) ou retirer un matériau osseux (22) mélangé au fluide (24) sur la surface intérieure (12) à partir du récipient (10) ;
**caractérisé en ce que** la surface extérieure (14) comprend en outre une surface d'étagement (32) adjacente à l'élément de verrouillage (20) conçu pour recevoir et mesurer le matériau osseux (22) pour l'entrée du matériau osseux (22) sur la surface intérieure (12) lorsque l'élément de verrouillage (20) est dans une position ouverte.

2. Récipient (10) selon la revendication 1, dans lequel l'orifice (26) comprend un canal (28) pour permettre à un fluide (24) d'entrer ou de sortir de la surface intérieure (12) du récipient (10), dans lequel, facultativement, le fluide (24) comprend un aspirat de moelle osseuse, une solution saline, de l'eau stérile, du sang pour injection, une solution saline tamponnée au phosphate, du dextrose, une solution lactée de Ringer, ou une combinaison de ceux-ci.

3. Récipient (10) selon la revendication 2, dans lequel (i) l'orifice (26) et/ou le canal (28) a un diaphragme (30) pour permettre à un fluide (24) d'entrer ou de sortir de la surface intérieure (12) du récipient (10) ; (ii) l'orifice (26) présente un raccord conçu pour venir en prise avec une seringue (50) ; ou (iii) l'orifice (26) et/ou le canal (28) peuvent être retirés du récipient (10).

4. Récipient (10) selon l'une des revendications 1 à 3, dans lequel l'élément de verrouillage (20) (i) enferme le matériau osseux (22) sur la surface intérieure (12) du récipient (10) lorsqu'il est dans une position fermée et permet l'entrée du matériau osseux (22) sur la surface intérieure (12) du récipient (10) lorsqu'il est dans une position ouverte ; (ii) comprend un premier élément de verrouillage (20) disposé sur la surface extérieure (14) et un élément de verrouillage correspondant (20) disposé sur la surface intérieure (12) pour permettre un joint entre la surface extérieure (14) et la surface intérieure (12) lorsque les éléments de verrouillage respectifs (20) sont mis en prise pour empêcher la perte de matériau osseux (22) ou de fluide (24) mélangé au matériau osseux (22) à partir du récipient (10) ; ou (iii) est refermable.

5. Récipient (10) selon l'une des revendications 1 à 4, dans lequel la surface d'étagement (32) comprend une ligne de déchirure (34) conçue pour retirer la surface d'étagement (32) du récipient (10).

6. Récipient (10) selon l'une des revendications 1 à 5, comprenant une couche supérieure (36) ayant la surface extérieure (14) et une couche inférieure (38) ayant la surface intérieure (12).

7. Récipient (10) selon la revendication 6, dans lequel (i) la couche supérieure (36) est pelable de la couche inférieure (38) et dans une direction vers l'extrémité distale (16) du récipient (10) pour permettre l'accès à n'importe quel matériau osseux (22) et/ou fluide (24) sur la surface intérieure (12) du récipient (10) ; (ii) la couche supérieure (36) et la couche inférieure (38) ont des parois latérales (44) qui forment un joint périmétrique (47) autour du récipient ; ou (iii) la couche supérieure (36) et la couche inférieure (38) ont des lignes de déchirure (34) adjacentes à l'orifice (26), les lignes de déchirure (34) conçues pour permettre un retrait permanent de l'orifice (26) de manière à former une ouverture (48) dans le récipient (10) pour distribuer du matériau osseux (22) et/ou du fluide (24) à partir de l'ouverture (48).

8. Récipient (10) selon l'une des revendications 1 à 7, dans lequel le récipient (10) comprend un matériau non poreux, un matériau transparent et/ou imperméable.

9. Récipient (10) selon l'une des revendications 1 à 8, dans lequel le matériau osseux (22) comprend de la céramique, du collagène, un os d'allogreffe, un os autogreffe, des fibres de matrice osseuse déminéralisée, une poudre osseuse déminéralisée, des copeaux osseux déminéralisés ou une combinaison de ceux-ci.

10. Récipient (10) selon l'une des revendications 1 à 9, dans lequel (i) l'extrémité proximale (18) du récipient (10) comprend un col (42) ; ou (ii) l'extrémité distale (16) du récipient (10) est conçue pour être enroulée et/ou pressée vers l'extrémité proximale (18) du récipient (10) pour distribuer du matériau osseux (22) et/ou du fluide (24) à partir du récipient (10).

11. Récipient (10) selon l'une des revendications 1 à 10, dans lequel le récipient (10) comprend des parois latérales flexibles (44) conçues pour permettre un mélange manuel de fluide (24) et du matériau osseux (22) sur la surface intérieure (12) par pression appliquée à la main.

12. Récipient (10) selon l'une des revendications 1 à 11, dans lequel l'orifice (26) est conçu pour recevoir une seringue (50) pour l'ajout de fluide (24) au récipient (10) ou une distribution précise de matériau osseux (22) à partir du récipient (10).

13. Procédé de mélange de matériau osseux (22), le procédé comprend la fourniture d'un récipient (10) pour mélanger un matériau osseux (22), le récipient (10) comprenant une surface intérieure (12) pour recevoir un matériau osseux (22) sur celle-ci et une surface extérieure (14) ayant une extrémité distale (16) et une extrémité proximale (18), l'extrémité distale (16) comprenant un élément de verrouillage (20) conçu pour enfermer le matériau osseux (22) sur la surface intérieure (12) du récipient (10), l'extrémité proximale (18) ayant un orifice (26) conçu pour recevoir un fluide (24) pour mélanger avec le matériau osseux (22) sur la surface intérieure (12) du récipient (10) ou retirer un matériau osseux (22) mélangé avec un fluide (24) sur la surface intérieure (12) du récipient (10) ; l'ouverture de l'élément de verrouillage (20) et l'ajout du matériau osseux (22) sur la surface intérieure (12) du récipient (10) ; la fermeture de l'élément de verrouillage (20) pour enfermer le matériau osseux (22) sur la surface intérieure (12) du récipient (10) ; et l'ajout de fluide (24) au récipient (10) par passage du fluide (24) à travers l'orifice (26) du récipient (10), dans lequel, éventuellement, le récipient (10) est stérile **caractérisé en ce que** le récipient (10) présente une surface d'étagement (32) adjacente à l'élément de verrouillage (20) conçu pour recevoir et mesurer un matériau osseux (22) ajouté au récipient (10) pour l'entrée du matériau osseux (22) sur la surface intérieure (12) lorsque l'élément de verrouillage (20) est dans une position ouverte.

14. Procédé selon la revendication 13, dans lequel le matériau osseux (22) comprend une céramique, un collagène, un os d'allogreffe, un os autogreffe, des fibres de matrice osseuse déminéralisée, une poudre osseuse déminéralisée, des copeaux osseux déminéralisés ou une combinaison de ceux-ci.

15. Procédé selon la revendication 13 ou 14, dans lequel le fluide (24) comprend un aspirat de moelle osseuse, une solution saline, de l'eau stérile, du sang pour injection, une solution saline tamponnée au phosphate, du dextrose, une solution lactée de Ringer, ou une combinaison de ceux-ci.

16. Procédé selon l'une des revendications 13 à 15, dans lequel le récipient (10) comprend un matériau non poreux, un matériau transparent et/ou imperméable.
